# EUROPÄISCHE PATENTSCHRIFT

(11) **EP 1 926 439 B1**
(45) Veröffentlichungstag und Bekanntmachung des Hinweises auf die Patenterteilung: **15.12.2010**
(21) Anmeldenummer: 06761250.7
(22) Anmeldetag: 02.08.2006
(51) Int. Cl.: A61B 17/11, A61F 2/06

(54) **VORRICHTUNG FÜR DIE OPERATIVE VERBINDUNG VON HOHLORGANEN, INSBESONDERE VON BLUTGEFÄSSEN**
DEVICE FOR CONNECTING HOLLOW ORGANS, ESPECIALLY BLOOD VESSELS, BY SURGERY
DISPOSITIF DE LIAISON FONCTIONNELLE D'ORGANES CREUX, NOTAMMENT DE VAISSEAUX SANGUINS

(30) Priorität: 03.08.2005 CH 12862005
(43) Veröffentlichungstag der Anmeldung: 04.06.2008
(73) Patentinhaber: King Faisal Specialist Hospital and Research Centre, Riyadh 11211 (SA)
(72) Erfinder: REDHA, Falah King Faisal Specialist Hospital & Research Centre, 11211 Riyadh (SA); KOBLER, Stefan, 4623 Neuendorf/SO (CH); ZUMBRUNN, Werner, 4132 Muttenz (CH); AL SHAIL, Essam Abdulaziz King Faisal Specialist Hospital & Research Centre, 11211 Riyadh (SA)
(74) Vertreter: Engelhard, Markus
(86) Internationale Anmeldenummer: PCT/CH2006/000399
(87) Internationale Veröffentlichungsnummer: WO 2007/014482

(56) Entgegenhaltungen:
- FR-A- 2 442 621
- US-A- 3 040 748
- US-A- 4 470 415
- US-A- 4 624 255
- US-A1- 2005 182 430

## Beschreibung

Die Erfindung betrifft eine Vorrichtung für die Anastomose von Hohlorganen.

Eine wichtige Aufgabe der Chirurgie ist die End-zu-End- Anastomose. Im Folgenden soll darunter vor allem die operative Verbindung von Blutgefässen verstanden werden.

Die weiter unten beschriebenen Techniken können prinzipiell aber auch für die Anastomose beliebiger Hohlorganstümpfe angewendet werden.

Bei der End-zu-End-Anastomose ist Nähen die am häufigsten verwendete Verbindungstechnik. Statt des zeitaufwendigen Nähens kann die Verbindung aber auch mit Hilfe von einem Fitting und Hülsen in einem Bruchteil der Zeit bewerkstelligt werden. Zu diesem Zweck wird über jedes der beiden Gefässenden eine vorzugsweise biologisch abbaubare Hülse geschoben. Ihr Innendurchmesser muss in etwa dem Aussendurchmesser der Gefässe entsprechen. Die Hülse wird jeweils so weit über das Gefäss geschoben, bis das Ende des Gefässes ein bisschen aus der Hülse ragt. Dann muss das Ende des Gefässes über das Ende der Hülse gestülpt werden, damit die Hülse festsitzt und die Innenseite des Gefässes, die Tunica intima, nach aussen zeigt. Die beiden Hülsen werden dann in ein Fitting geschoben und in diesem so fixiert, dass sich die Innenseiten beider Gefässe über den ganzen Umfang berühren. Dies ist eine Voraussetzung dafür, dass die beiden Gefässenden zusammenwachsen können.

Bei diesem Prozedere kann die Arbeit des Chirurgen mit verschiedenen Hilfsmitteln unterstützt werden, zum Beispiel mit Vorrichtungen, welche die Enden der Blutgefässe halten und abklemmen, die Hülsen halten und helfen, diese axial aufeinander auszurichten, die das Zusammenschieben der Hülsen erleichtern oder mit einer Vorrichtung, die das Umstülpen der Blutgefässe über die Enden der Hülsen unterstützt und erleichtert.

Viele Hilfsmittel für die Anastomose von Gefässen sind schon seit Jahrzehnten bekannt. In US 4,474,181 und US 4,624,255 wird eine Vorrichtung vorgestellt, die im wesentlichen aus einem Ring besteht, dessen Durchmesser grösser als der Aussendurchmesser des Blutgefässes ist. Die zu verbindenden Gefässenden werden zuerst an mindestens drei Stellen zusammengenäht. Dann wird der Ring über die Nahtstelle geschoben. Mit Hilfe der Fäden der Nähte wird das Gefäss auf den Durchmesser des Rings ausgeweitet und in dieser Stellung am Ring fixiert. Dadurch werden die Innenseiten beider Gefässe miteinander in Kontakt gebracht. In US 4,016,883 und US 4,165,747 finden sich Beispiele für Klemmen, vor allem für Blutgefässe mit kleinem Durchmesser. In US 4,165,747 werden überdies Klemmen vorgeschlagen, die auf einer gemeinsamen Schiene verschiebbar angeordnet sind. Auf diese Art und Weise können die Blutgefässe gleichzeitig abgeklemmt und die gegenseitige Lage fixiert werden; dies erleichtert das Zusammennähen.

Weitere Vorrichtungen, welche die gegenseitige axiale Ausrichtung der Gefässenden und der Hülsen und des Fittings gewährleisten, sind in US 1,151,300, US 2,940,451 und US 3,048,177 beschrieben. In allen Fällen muss vor dem Verbinden mindestens ein Gefässende um eine Hülse umgestülpt werden, damit sich nach dem Zusammenfügen die Innenseiten beider Gefässe berühren.

Im Gegensatz zu den oben erwähnten Patentschriften ist in US 2,453,056 beschrieben, wie das Umstülpen der Gefässenden bewerkstelligt wird: mit Hilfe von Pinzetten. Es ist bekannt, dass das Umstülpen von Gefässen mit Hilfe mehrerer Pinzetten sehr schwierig ist und unter Umständen mehr als eine Person beansprucht. Selbst bei einer Unterstützung des Chirurgen durch eine mechanische Vorrichtung, die mehrere Pinzetten oder Greifer in geeigneter Weise halten und führen kann, bleibt die Gefahr bestehen, dass die Gefässenden bei den Manipulationen beschädigt werden.

US 2,453,056 beweist, dass das Verwenden von Hülsen oder Fittings als Hilfsmittel für die Anastomose schon sehr lange bekannt ist. Auch in den Patentschriften US 3,221,746, US 3,254,650, US 3,254,651, US 3,774,615, US 4,366,819 oder US 2004/0199189 A1 wird die Verwendung von Fittings vorgeschlagen.

Auch für das Umstülpen der Gefässenden um das Ende einer Hülse sind verschiedene Hilfsmittel entwickelt worden. In GB 1413191 werden mehrere Stacheln vorgeschlagen, die aussen an der Hülse angebracht und in axialer Richtung bewegbar sind. Sie haben nach aussen gerichteten Widerhaken und ragen ein bisschen über das Hülsenende hinaus. Der Rand des Gefässes, das vorgängig durch die Hülse geschoben worden ist, wird mit Hilfe von Pinzetten auf die Widerhaken aufgezogen respektive aufgestochen. Dann werden die Stacheln zurückgezogen. Dergestalt wird das Gefässende über das Ende der Hülse gezogen.

In US 2,940,452 wird die Verwendung einer Membran aus einem Elastomer vorgeschlagen. Sie ist über das Ende eines Rohres gespannt. Durch das Innere des Rohres wird nun ein zylindrischer Kolben mit konischem Ende vorgeschoben, bis die Membran eine konische Ausbuchtung hat. In dieser Lage wird der Kolben mit der darüber gespannten Membran in das eine Hülse überragende Ende des Blutgefässes geschoben und dann gegen die Hülse gepresst. Anschliessend wird das äussere Rohr in Richtung des Gefässes geschoben. Dabei legt sich die Membran um das Ende der Hülse - und mit ihr der überstehende Teil des Blutgefässes.

In US 3,180,337 ist eine ähnliche Vorrichtung, aber ohne Membran, beschrieben. Es wird ebenfalls ein zylindrischer Kolben mit konischem Ende verwendet. Der zylindrische Teil des Kolbens wird von einem axial verschiebbaren Rohrstück aus elastischem Material umschlossen. Zuerst wird das konische Ende des Kolbens in das eine Hülse überragende Ende des Blutgefässes geschoben und dann gegen die Hülse gepresst. Anschliessend wird das erwähnte Rohrstück über den Kolben in Richtung Blutgefäss geschoben. Zuerst staucht das Rohrstück das überstehende Ende des Blutgefässes, dann weitet es sich zusammen mit dem Ende des Blutgefässes auf, und letztlich stülpen sich beide über das Hülsenende.

In US 2,779,996 wird als Hilfsmittel ein elastischer Ring beschrieben, der in das Innere des überstehenden Teils des Gefässendes geschoben wird. Nach dem Einführen wird der Ring - und damit auch der überstehende Teil des Gefässes - mit Hilfe von Druckluft ausgeweitet, und auf diese Art und Weise wird der überstehende Teil des Gefässendes um die Hülse gestülpt.

Die Vorrichtung gemäss US 3,040,748 ähnelt den Hilfseinrichtungen von US 2,940,452 und US 3,180,337. Sie verwendet in bekannter Art und Weise einen zylindrischen Kolben mit konischem Ende. Hinter dem konischen Kopf ist ein dünnes Rohrstück aus einem elastischen Material aufgezogen. Das hintere Ende dieses Rohres hat einen Wulst. Er wird von einem weiteren, in axialer Richtung verschiebbaren Rohr gehalten und ist bereits ausgeweitet. In bekannter Manier wird nun der Kolben mit dem konischen Ende in den überstehenden Teil des Blutgefässes geschoben und dann gegen die Hülse gepresst. Unterstützt wird dieser Vorgang durch ein Fluid, das durch die Spitze des konischen Kolbenkopfes in das abgeklemmte Gefäss gepumpt wird. Das Fluid strömt zwischen Gefäss und Kolben sowie Rohrstück aus elastischem Material durch und erleichtert so das Aufschieben des Gefässendes auf das Rohrstück. Wenn das Aufschieben beendet ist, wird der Flüssigkeitsstrom unterbrochen. Dann wird das äussere, verschiebbare Rohr in Richtung der Hülse geschoben. Es nimmt das elastische Rohrstück - und damit das übergeschobene Gefässende - mit und stülpt beide um die Hülse.

In US 4,055,186 wird ein Druckknopfsystem für das Zusammenfügen zweier Darmteile geschildert. An beiden Teilen des Druckknopfes ist ein konzentrischer Ring befestigt, der in axialer Richtung federnd gelagert ist. Die Ringe haben einen Aussendurchmesser, der etwas kleiner als der Innendurchmesser der zu verbindenden Darmteile ist. Die Druckknopfteile mit den Ringen werden in die Enden der Darmabschnitte geschoben und diese anschliessend nach innen um die Ringe gestülpt; wie dies zu bewerkstelligen ist, wird nicht beschrieben. Anschliessend werden die Druckknopfteile zusammengefügt und eingerastet. Dank der federnden Ringe werden die beiden Darmabschnitte mit einer definierten Kraft gegeneinander gepresst.

US 4,470,415 offenbart eine Vorrichtung und ein Verfahren zur nahtlosen chirurgischen Anastomose. Eine wärme-schrumpfbare Hülse wird um zwei röhrenförmige Elemente positioniert, die anastomosiert werden sollen und wird dann geschrumpft, sodass sie mit den beiden röhrenförmigen Elementen in Eingriff steht und diese in einer anastomotischen Beziehung hält. Die Enden der röhrenförmigen Elemente werden über starre oder halbstarre Ringe umgestülpt, die an den Enden der röhrenförmigen Elemente angeordnet sind.

Alle vorgenannten Vorrichtungen für die von Blutgefässen weisen gewisse Mängel in der Handhabung oder in ihrer Funktion auf. Der Erfindung liegt deshalb die Aufgabe zugrunde eine verbesserte Vorrichtung zur Anastomose ohne die beim Stand der Technik vorhandenen Mängel zu finden.

Erfindungsgemäss wird diese Aufgabe gelöst durch eine Vorrichtung, die sich durch die im Anspruch 1 angegebenen Merkmale auszeichnet.

Zunächst wird eine Umstülpvorrichtung beschrieben, die sich durch kleinsten Raumbedarf und einfachste Bedienung auszeichnet. Der erste Vorbereitungsschritt für das Umstülpen entspricht dem üblichen Vorgehen: eine zylindrische Hülse wird so weit über ein Gefässende geschoben, bis dieses um ein bestimmtes Mass aus der Hülse ragt. Der überstehende Teil des Gefässes sollte gerade so lang sein, dass die Hülse nach dem Umstülpen des Gefässendes genügend festsitzt. Vorausgesetzt wird im Weiteren, dass das Gefäss weiter hinten abgeklemmt ist und dass sein Ende sauber bearbeitet ist, zum Beispiel durch einen Schnitt senkrecht zur Längsachse des Gefässes.

Anschliessend wird eine rotationssymmetrische Hilfseinrichtung von aussen her, axial ausgerichtet, gegen die Öffnung des Gefässes geschoben. Der Vorderteil dieser Hilfseinrichtungbesteht aus einem vorne abgerundeten Zapfen, dessen Aussendurchmesser etwa den Innendurchmesser des Gefässes hat. Der Zapfen ist zumindest so lang wie der über die Hülse hinausragende Teil des Gefässes und hat eine an seinem vorderen Ende mündende axiale Bohrung. Durch sie wird während des Umstülpvorgangs ein körperverträgliches Fluid unter Druck nach aussen gepumpt.

Der hintere Teil der Hilfseinrichtung ist zylindrisch, und sein Durchmesser ist grösser als derjenige des Zapfens. Die dem Gefäss zugewandte Stirnseite dieses Hinterteils ist bei vorteilhaften Ausführungsformen konkav.

Wenn der vordere Teil des Zapfens in das Gefäss eintritt, baut sich im Gefäss ein Druck auf. Da das Gefäss weiter hinten abgeklemmt ist, muss das durch den Zapfen gepumpte Fluid zwischen der Innenseite des Gefässes und dem Zapfen nach aussen strömen; es bildet auf diese Art und Weise einen Schmierfilm. Die Reibung zwischen Gefäss und Zapfen wird dadurch sehr klein, und der Zapfen kann folglich gut in das Gefäss eingeführt werden.

Der Druckaufbau im Gefäss bewirkt, dass es gegen die Innenseite der besagten Hülse gepresst wird und eine Haftreibung entsteht. Durch eine geeignete Struktur der Hülseninnenseite kann die Haftreibung zusätzlich vergrössert werden, so dass das Gefäss trotz des Druckes in seinem Innern nicht nach hinten ausweichen kann, sondern an der Hülseninnenseite haftet.

Solange das überstehende Ende des Gefässes die Stirnseite des Hinterteils der Hilfseinrichtung noch nicht erreicht hat, geschieht nichts weiter. Wenn aber das Ende des Gefässes auf diese Stirnfläche trifft, dann wird es in Richtung der Hülse zurückgestossen. Derjenige Teil des Gefässes, der sich im Innern der Hülse befindet, kann aber wegen der erwähnten Haftreibung nicht zurückweichen.

Folglich wird der über die Hülse hinausragende Teil des Gefässes gestaucht. Gleichzeitig erhöht sich der Druck im Fluid. Die Folge ist, dass sich der überstehende Teil des Gefässes ausweitet. Da das Fluid zwischen dem Ende des Gefässes und der Stirnseite des Hinterteils der Hilfseinrichtung durchströmen muss, gibt es auch hier keine Haft- oder Gleitreibung, die das Ausweiten des Gefässes behindern könnte.

Das Stauchen und Ausweiten des überstehenden Teils des Gefässes hat zur Folge, dass sich die Aussenseite dieses Gefässteils um das Ende der Hülse zu legen beginnt. Mit dem Fortschreiten dieses Prozesses legt sich ein immer grösserer Teil des Gefässes um das Ende der Hülse. Letztlich gerät das Ende des Gefässes in einen indifferenten Gleichgewichtszustand, und von da aus geht es unter einer bestimmten Voraussetzung ohne äusseres Zutun in eine stabile Lage - vollständig umgestülpt. Die Voraussetzung ist, dass das überstehende Ende des Gefässes ursprünglich nicht zu lang war.

Im folgenden wird anhand der beiliegenden Zeichnungen ein bevorzugtes Ausführungsbeispiel der Erfindung beschrieben. Es zeigen
Fig. 1 eine Umstülpvorrichtung im Zustand, bevor der Vorderteil der sich Richtung Gefässöffnung bewegenden Hilfseinrichtung in das Gefäss eingetreten ist
Fig. 2 den Moment, in dem das Ende des Gefässes erstmals an den Hinterteil der Hilfseinrichtung stösst
Fig. 3 das gestauchte und ausgeweitete Ende des Gefässes
Fig. 4 den Moment, in dem das Ende des Gefässes einen indifferenten Gleichgewichtszustand erreicht hat
Fig. 5 eine Darstellung des vollständig umgestülpten Gefässendes am Ende des Umstülpprozesses
Fig. 6 ein System, das aus zwei Hülsen und einem Fitting für das Verbinden von zwei Gefässenden besteht
Fig. 7 eine komplette Vorrichtung mit Haltevorrichtungen für Adernklemmen, Haltevorrichtungen für Fittings und Hülsen, Platz sparender Hilfseinrichtung für das Umstülpen der Enden der Blutgefässe und ein Schienensystem, auf dem die Haltevorrichtungen seitlich verschoben, rotiert, in ihrer Lage fixiert und zum Teil aufgesteckt und demontiert werden können.

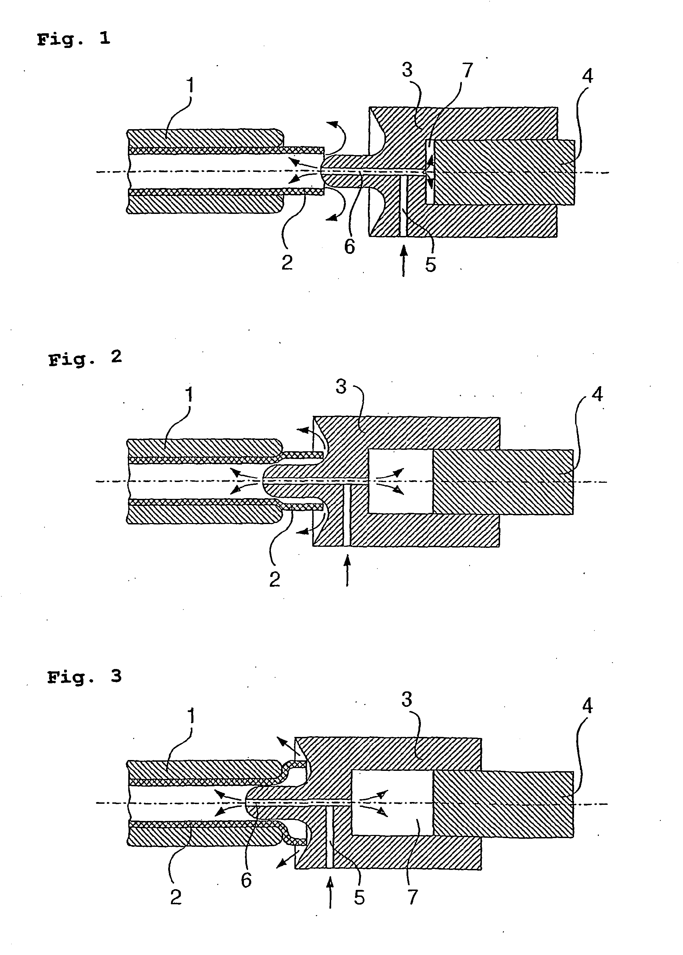

In Fig. 1 sind die wesentlichen Teile einer infolge ihrer Kompakt- und Einfachheit für das Gesamtsystem geeigneten Umstülpvorrichtung dargestellt: Eine über ein Gefäss (2) geschobene Hülse (1), eine bewegliche Hilfseinrichtung (3) und ein Kolben (4), der in eine zylindrische Öffnung (7) des Hinterteils der Hilfseinrichtung (3) hineinragt. Alle Teile sind axial ausgerichtet. Die Hülse (1), das Gefäss (2) und der Kolben (4) sind feststehende Teile, während die Hilfseinrichtung (3), auf dem Kolben (4) gelagert, in axialer Richtung beweglich ist. Die Hilfseinrichtung (3) hat eine Öffnung (5), in die ein körperverträgliches Fluid gepresst wird. Das Fluid strömt durch einen axialen Kanal (6) sowohl ins Innere des Gefässes (2) als auch in den Zylinderraum (7). Der Kolben (4) wird verdrängt, und damit setzt sich die Hilfseinrichtung (3) in Richtung des Gefässes (2) in Bewegung. Da vorausgesetzt wird, dass das Gefäss (2) in oder hinter der Hülse (1) abgeklemmt ist, muss das Fluid zwischen Gefäss (2) und Vorderteil der Hilfseinrichtung (3) nach aussen strömen. Damit ist gewährleistet, dass die Reibung zwischen Gefäss (2) und Vorderteil der Hilfseinrichtung (3) gering ist und der Vorderteil der Hilfseinrichtung (3) leicht in das Gefäss (2) eindringen kann.

In Fig. 2 ist der Moment dargestellt, in dem das Ende des Gefässes (2) an die Stirnseite des hinteren Teils der Hilfseinrichtung (3) stösst. Der Druck im Innern des Gefässes (2) erhöht sich. Dadurch wird das Gefäss (2) an die Innenseite der Hülse (1) gepresst, und die Haftreibung nimmt zu. Deshalb kann das Gefäss (2) nicht zurück gleiten, wenn jetzt der Hinterteil der Hilfseinrichtung (3) das Ende des Gefässes (2) zu stauchen beginnt. Der Druck im Fluid und das Stauchen bewirken, dass das Ende des Gefässes (2) ausgeweitet wird und dass es beginnt, sich um das Ende der Hülse (1) zu schmiegen.

In Fig. 3 wird der Zustand in einem späteren Zeitpunkt gezeigt. Das Ende des Gefässes (2) ist noch mehr gestaucht und hat sich noch mehr um das Ende der Hülse (1) gelegt.

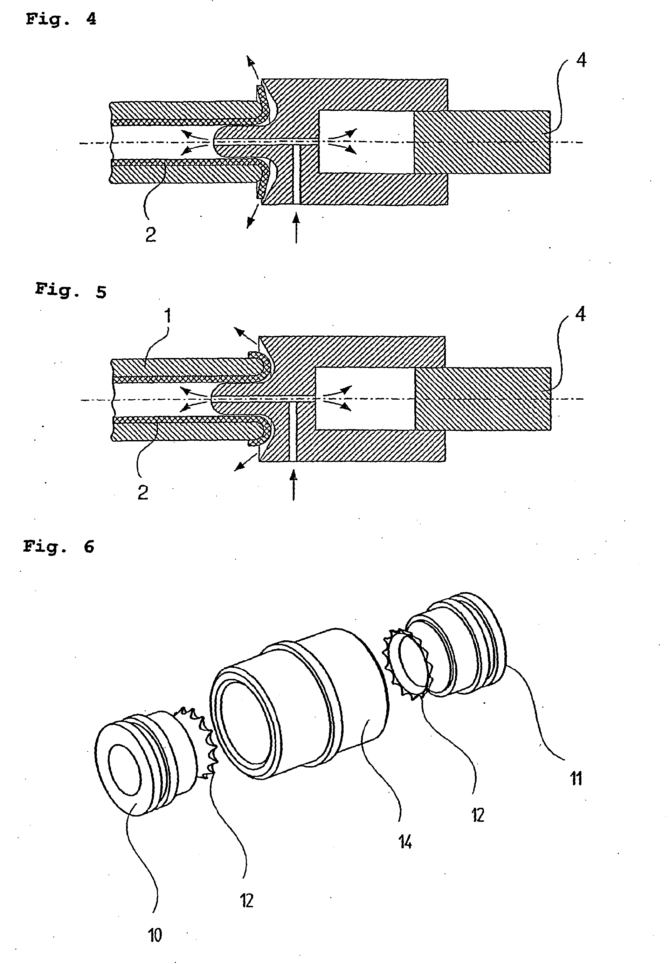

In Fig. 4 ist das Ende des Gefässes (2) nach noch stärkerem Stauchen und Ausweiten im indifferenten Gleichgewichtszustand dargestellt.

Nachdem das Ende des Gefässes (2) von selbst in eine neue Gleichgewichtslage gelangt ist, schmiegt es sich - wie Fig. 5 zeigt - vollständig um das Ende der Hülse (1).

Es sind auch Ausführungsformen denkbar, die ohne Kolben (4) arbeiten. Die Hilfseinrichtung (3) sollte aber auch in diesem Fall axial verschiebbar gelagert sein, damit das Einführen der Hilfseinrichtung (3) in das Gefäss (2) leichter gelingt. Der axiale Vorschub wird dann zum Beispiel manuell oder mit Federkraft bewerkstelligt.

In Fig. 6 sind für die End-zu-End-Anastomose von Hohlorganen geeignete Hülsen und ein Fitting dargestellt. Die Verbindung kommt folgendermassen zustande: Je ein Ende der zu verbindenden Hohlorgane wird in je eine Hülse (10, 11) eingeschoben - und zwar so weit, bis die Hohlorgane ein bisschen über die Enden (12) der Hülsen hinausragen. Dann werden die Enden der Hohlorgane um die Enden (12) der Hülsen gestülpt. Danach werden die Hülsen (10, 11) in das Fitting (14) geschoben, bis sich die umgestülpten Teile der Hohlorgane berühren. In dieser Lage werden die beiden Hülsen (10, 11) dann fixiert.

Die Hülsen (10) und (11) sind identisch aufgebaut. Ihr Innendurchmesser entspricht in etwa dem Aussendurchmesser der zu verbindenden Hohlorgane. Die Enden (12) der Hülsen sind so geformt, dass der Aussendurchmesser der umgestülpten Hohlorgane gleich oder kleiner als der Innendurchmesser des Fittings (14) ist. Des Weiteren können die Enden (12) der Hülsen Spitzen, Zacken oder Widerhaken haben, damit sich die umgestülpten Hohlorgane nicht mehr lösen können. Damit die Verbindung hergestellt werden kann, müssen weitere Voraussetzungen erfüllt sein: Die Passung aus Innendurchmesser des Fittings (14) und Aussendurchmesser der Hülsen (10,11) kann entweder ein Press-, Fest-, Haft- oder ein Gleitsitz sein. Im ersten Fall muss der Press-, Fest- oder Haftsitz so bemessen sein, dass nach dem Einschieben der Hülsen (10, 11) in das Fitting (14) jene so festsitzen, dass sie sich bei der erwarteten Zugbeanspruchung der Hohlorgane nicht lösen können.

Im zweiten Fall müssen spezielle Fixierelemente vorgesehen werden. Zum Beispiel kann das Fitting (14) auf seiner Innenseite zwei ringförmige Nuten und können die Hülsen (10, 11) je einen entsprechenden Wulst haben. Der Abstand der beiden Nuten ist so gewählt, dass sich die Innenseiten der umgestülpten Hohlorgane gerade berühren, wenn die Wülste der Hülsen (10, 11) in die Nuten des Fittings (14) eingerastet sind. Damit die Hülsen (10, 11) mit ihren Wülsten überhaupt in das Fitting (14) eingeschoben werden und einrasten können, muss diese federnd radial dehnbar sein.

Vorteilhaft ist es auch, wenn das Fitting (14) in der Mitte geteilt ist und die beiden Teile mit einer axial wirkenden Feder verbunden sind oder wenn der mittlere Teil des Fittings (14) als eine solche Feder ausgebildet ist. Die Federkonstante wird so gewählt, dass nach dem Einpressen oder Einrasten der Hülsen (10, 11) in dem Fitting (14) die beiden umgestülpten Hohlorgan-Endstücke mit einer definierten Kraft zusammengepresst werden.

Die Hülsen und das Fitting werden mit Vorteil aus biologisch abbaubarem Material hergestellt, und die Innenseiten der Hülsen (10, 11) haben mit Vorteil eine Oberflächentextur; sie erhöht während des weiter oben geschilderten Umstülpprozesses die Haftreibung zwischen Hülse bzw. Fitting und Hohlorgan.

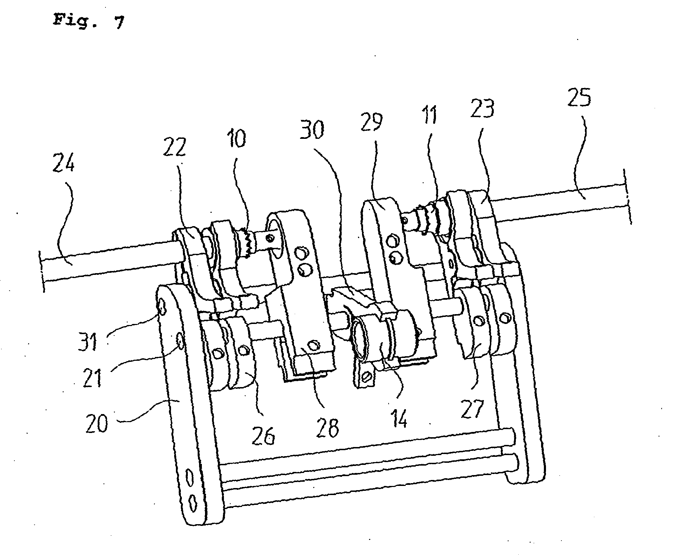

In Fig. 7 ist ein komplettes System für die End-zu-End- Anastomose von Hohlorganen dargestellt. Die Basis des Systems ist ein starrer Rahmen (20). Auf einer Welle (21) sind verschiedene Halte- bzw. Klemmvorrichtungen dreh- und verschiebbar gelagert. Damit sie auf einfache Art und Weise axial aufeinander ausgerichtet werden können, ist die Traverse (31) als Anschlag konzipiert. Wenn die Haltevorrichtungen bis an diesen Anschlag nach hinten gedreht werden, sind sie axial ausgerichtet.

Ganz links und ganz rechts ist je eine Klemmvorrichtung (22, 23) montiert. Sie dienen dazu, die beiden zu verbindenden Hohlorgane (24, 25) abzuklemmen und gleichzeitig festzuhalten. Direkt neben jeder Klemmvorrichtung ist je eine Vorrichtung (26, 27) für das Festhalten der Hülsen (10, 11). Rechts neben der einen Haltevorrichtung (26) für die eine Hülse (10) befindet sich eine Umstülpvorrichtung (28), und links neben der anderen Haltevorrichtung (27) für die andere Hülse (11) befindet sich eine weitere Umstülpvorrichtung (29). Diese Umstülpvorrichtungen sind auf die Welle (21) aufsteckbar. Nach dem Umstülpen der Hohlorgane werden sie nicht mehr gebraucht und können entfernt werden.

In der Mitte des Systems befindet sich eine weitere Haltevorrichtung (30) für das Fitting (14). Diese verschiebbare Haltevorrichtung wird nach dem Drehen bis an den Anschlag (31) zuerst gegen die Haltevorrichtung (26) geschoben. Dabei wird das Fitting (14) über die Hülse (10) geschoben und die beiden Teile gegenseitig fixiert. Anschliessend wird das Hohlorgan (24) mit der Hülse (10) aus den Halterungen (22) und (26) gelöst und zusammen mit der Haltevorrichtung (30) gegen die Haltevorrichtung (27) geschoben. Dabei wird das Fitting (14) über die Hülse (11) geschoben und die beiden Teile gegenseitig fixiert.

Nach der zweiseitigen Verbindung ist die End-zu-End- Verbindung hergestellt, und die zusammengekoppelten Hohlorgane mit ihren Hülsen und dem Fitting (10, 11, 14) können aus den Haltevorrichtungen (23, 27, 30) ausgeklinkt werden.

## Patentansprüche

1. Vorrichtung für die Anastomose von Hohlorganen, wobei die besagte Vorrichtung mindestens die folgenden Hilfseinrichtungen umfasst:
- zwei Hülsen (10, 11), die so über die beiden zu verbindenden Hohlorganstümpfe (24, 25) geschoben werden können, dass diese um ein bestimmtes Mass über die Hülsenenden hinaus ragen,
- eine oder mehrere Einrichtungen (28), die das Umstülpen der Enden der Hohlorgane um die Enden der besagten Hülsen unterstützen und erleichtern,
- ein Fitting (14), in das die beiden Hülsen mit den umgestülpten Hohlorganen eingeschoben und so fixiert werden können, dass sich die Innenseiten der Hohlorgane berühren,
- ein System von Haltevorrichtungen, auf einer oder mehreren Schienen montiert, mit einer oder mehreren der folgenden Eigenschaften: seitlich verschiebbar, abnehmbar, aufsteckbar, drehbar, in ihrer Lage fixierbar,
- wobei diese Haltevorrichtungen die besagten Hülsen und das besagte Fitting aufnehmen können, die eine oder mehrene Umstülpeinrichtungen halten können, allenfalls Klemmen für das Halten und Abklemmen der Hohlorgane halten können und mittels einer Hilfseinrichtung axial aufeinander ausgerichtet werden können.

2. Vorrichtung nach Anspruch 1, **dadurch gekennzeichnet, dass** die eine oder mehrere Umstülpeinrichtung (28) eine im wesentlichen rotationssymmetrische Hilfseinrichtung ist, deren Vorderteil aus einem Zapfen besteht, dessen Durchmesser etwa dem Innendurchmesser des Hohlorgans entspricht, deren Hinterteil einen Durchmesser hat, der grösser als derjenige des Vorderteils ist, dass während des Umstülpvorgangs aus dem besagtem Zapfen über einen Auslass oder über mehrere Auslässe ein Fluid unter Druck herausgepresst wird, und zum Zwecke des Umstülpens diese Hilfseinrichtung mit dem Vorderteil voran so weit in die Öffnung des in oder hinter der besagten Hülse abgeklemmten Hohlorgans geschoben wird, bis sich das Ende des Hohlorgans um das Ende der besagten Hülse gelegt hat.

3. Vorrichtung nach Anspruch 2, **dadurch gekennzeichnet, dass** die der Öffnung des Hohlorgans zugewandte Stirnseite des hinteren Teils der besagten Hilfseinrichtung (28) einen konkaven Teil aufweist.

4. Vorrichtung nach einem der Ansprüche 2 - 3, **dadurch gekennzeichnet, dass** der hintere Teil der besagten Hilfseinrichtung (28) für den Vortrieb einen Zylinder (3) und einen Kolben (4) enthält, der von der gleichen Fluidquelle wie die Auslässe der besagten Hilfseinrichtung gespeist wird.

5. Vorrichtung nach Anspruch 1, **dadurch gekennzeichnet, dass** die Passung der beiden besagten Hülsen (10, 11) für die Aufnahme der Hohlorganstümpfe (24, 25) und des Fittings (14) für die Aufnahme dieser beiden Hülsen einen Press-, Fest- oder Haftsitz ermöglicht.

6. Vorrichtung nach Anspruch 1, **dadurch gekennzeichnet, dass** jede der beiden besagten Hülsen (10, 11) für die Aufnahme der Hohlorganstümpfe (24, 25) mindestens eine über den ganzen äusseren Umfang verlaufende Nut oder einen Wulst aufweist, dass das besagte Fitting für die Aufnahme der beiden Hülsen mindestens zwei über den ganzen inneren Umfang verlaufende Wülste oder Nuten aufweist, in welche die Nuten oder die Wülste der beiden Hülsen einrasten können.

7. Vorrichtung nach Anspruch 6, **dadurch gekennzeichnet, dass** das Fitting (14) für die Aufnahme der beiden Hülsen (10, 11) längs ganz oder teilweise geschlitzt ist, um das Einführen der beiden Hülsen zu ermöglichen oder zu erleichtern.

8. Vorrichtung nach einem der Ansprüche 6 - 7, **dadurch gekennzeichnet, dass** das besagte Fitting (14) für die Aufnahme der beiden Hülsen (10, 11) in der Mitte geteilt ist, die Teile aber über einen axial federnden Mittelteil verbunden sind, damit nach dem Einrasten der beiden Hülsen eine vordefinierte Kraft auf die umgestülpten Hohlorganstümpfe (24, 25) ausgeübt wird.

9. Vorrichtung nach Anspruch 1, **dadurch gekennzeichnet, dass** die Innenseite der besagten Hülsen (10, 11) für die Aufnahme der Hohlorganstümpfe (24, 25) eine Oberflächenstruktur haben, welche die Haftreibung vergrössert, wenn das Hohlorgan gegen die Innenseite gepresst wird, aber die gleichzeitig das Einführen des Hohlorgans in die jeweilige Hülse nicht wesentlich behindert.

## Claims

1. Device for connecting hollow organs, which device comprises at least the following elements:
- two fittings (10,11), that can be pushed over the two hollow organ ends that are to be connected (24,25) to such an extent as to project over the fitting ends by a defined extent,
- one or more devices (28) that support and facilitate the turning up of the ends of the hollow organs around the ends of the said fittings, an additional fitting (14) into which the two fittings are inserted with the turned-up hollow organs are and can be secured in such a way that the inner sides of the hollow organs are in contact with each other,
- a system of holding devices, mounted on one or more slide rails, with one or more of the following properties: laterally displaceable, detachable, stackable, capable of being rotated, capable of being secured in position,
- where these holding devices can hold the said fitting turn-up device, and clamps for holding the device and the said additional fitting in place can be attached to them; these clamps can hold and clamp off one or more of the hollow organs and can be placed in axial alignment to one another by means of an accessory.

2. Device according to claim 1, **characterised by** the fact that one or more of the turn-up devices (28) is a fundamentally rotation-symmetrical accessory, the front part of which comprises a cone, the diameter of which is similar to the interior diameter of the hollow organ, the rear part of which has a diameter greater than that of the front part, that during the turn-up process fluid is expelled under pressure from the said cone via one or more outlets, and in order to achieve turn-up the front part of this accessory is pushed into the opening of or behind the said fitting until the end of the hollow organ surrounds the end of the said fitting.

3. Device according to claim 2, **characterised by** the fact that the front side of the rear part of the said accessory (28) facing towards the opening of the hollow organ has a concave part.

4. Device according to one of claims 2 - 3, **characterised by** the fact that the rear part of the said accessory (28) contains a cylinder (3) and a piston (4) to drive the accessory, powered by the same fluid source as the outlets of the said accessory.

5. Device according to claim 1, **characterised by** the fact that the clearance of the two said fittings (10, 11) is sufficient to allow the hollow organ ends (24, 25) to fit snugly, and that the clearance of the additional fitting (14) is sufficient to allow both these fittings to fit snugly.

6. Device according to claim 1, **characterised by** the fact that each of the two said fittings (10, 11) has at least one groove or beading running along the entire outer perimeter to allow the hollow organs ends (24, 25) to penetrate, and that the said additional fitting has at least two beadings or grooves running along the entire inner perimeter to allow the two grooves or beadings of the fittings to penetrate and click into place.

7. Device according to claim 6, **characterised by** the fact that the additional fitting (14) is slotted along all or part of its length in order to permit or facilitate the introduction of the two fittings (10, 11).

8. Device according to one of claims 6 - 7 , **characterised by** the fact that the said additional fitting (14) which the two fittings (10, 11) enter is divided in the middle, with the sections being connected via an axially elastic central part, so that once the two fittings have clicked into place a predefined force is applied around the turned-up hollow organ ends (24,25).

9. Device according to claim 1, **characterised by** the fact that the interior of the said fittings (10, 11) which the hollow organ ends (24, 25) enter has a surface structure that increases adhesion when the hollow organ is pressed against the interior, but that at the same time does not significantly impede the introduction of the hollow organ into the relevant fitting.

## Revendications

1. Dispositif pour l'anastomose d'organes creux, dans lequel ledit dispositif comporte au moins les dispositifs auxiliaires suivants :
- deux douilles (10, 11) qui peuvent être poussées au-dessus des deux moignons d'organes creux à relier (24, 25) de telle sorte que ceux-ci font saillie dans une certaine mesure au-delà des extrémités de douilles,
- un ou plusieurs dispositifs (28) qui aident et facilitent le retournement des extrémités des organes creux autour des extrémités desdites douilles,
- un raccord (14) dans lequel les deux douilles pénètrent avec les organes creux retournés et peuvent être fixées de telle sorte que les côtés intérieurs des organes creux sont en contact l'un avec l'autre,
- un système de dispositifs de maintien, monté sur un ou plusieurs rails, ayant une ou plusieurs des propriétés suivantes : latéralement mobile, amovible, enfichable, rotatif, fixable dans sa position,
- ces dispositifs de maintien pouvant recevoir lesdites douilles et ledit raccord, peuvent maintenir un ou plusieurs dispositifs de retournement, éventuellement maintenir des pinces pour maintenir et détacher les organes creux, et être alignés axialement l'un avec l'autre au moyen d'un dispositif auxiliaire.

2. Dispositif selon la revendication 1, **caractérisé en ce que** le ou les dispositifs de retournement (28) sont un dispositif auxiliaire sensiblement symétrique en rotation dont la partie avant est constituée d'un tourillon ayant un diamètre correspondant approximativement au diamètre intérieur de l'organe creux, dont la partie arrière a un diamètre qui est plus grand que celui de la partie avant, **en ce que** lors du processus de retournement, un fluide sous pression est évacué par pression hors dudit tourillon par une sortie ou par plusieurs sorties, et aux fins du retournement, ce dispositif auxiliaire est poussé par la partie avant dans l'ouverture de l'organe creux détaché dans ou derrière ladite douille, jusqu'à ce que l'extrémité de l'organe creux se soit placée autour de l'extrémité de ladite douille.

3. Dispositif selon la revendication 2, **caractérisé en ce que** le côté avant de la partie arrière dudit dispositif auxiliaire (28), dirigé vers l'ouverture de l'organe creux, a une partie concave.

4. Dispositif selon l'une quelconque des revendications 2 à 3, **caractérisé en ce que** la partie arrière dudit dispositif auxiliaire (28) contient, à des fins d'entraînement, un cylindre (3) et un piston (4) qui est alimenté par la même source de fluide que les sorties dudit dispositif auxiliaire.

5. Dispositif selon la revendication 1, **caractérisé en ce que** l'ajustement desdites deux douilles (10, 11) pour recevoir les moignons d'organes creux (24, 25) et l'ajustement du raccord (14) pour recevoir ces deux douilles permet un ajustement par pression, un ajustement serré ou un ajustement par collage.

6. Dispositif selon la revendication 1, **caractérisé en ce que** chacune desdites deux douilles (10, 11) destinées à recevoir les moignons d'organes creux (24, 25) comporte au moins une gorge ou un bourrelet s'étendant sur la circonférence extérieure complète, **en ce que** ledit raccord destiné à recevoir les deux douilles comporte au moins deux bourrelets ou gorges s'étendant sur la circonférence intérieure complète, dans lesquels les gorges ou les bourrelets des deux douilles peuvent s'enclencher.

7. Dispositif selon la revendication 6, **caractérisé en ce que** le raccord (14) destiné à recevoir des deux douilles (10, 11) est fendu entièrement ou partiellement dans la direction longitudinale afin de permettre ou de faciliter l'insertion des deux douilles.

8. Dispositif selon l'une quelconque des revendications 6 à 7, **caractérisé en ce que** ledit raccord (14) destiné à recevoir les deux douilles (10, 11) est divisé au centre mais les parties sont reliées par une partie centrale axialement élastique afin que, après l'enclenchement des deux douilles, une force prédéfinie soit exercée sur les moignons d'organes creux retournés (24, 25).

9. Dispositif selon la revendication 1, **caractérisé en ce que** le côté intérieur desdites douilles (10, 11) destinées à recevoir les moignons d'organes creux (24, 25) a une structure de surface qui augmente le frottement statique lorsque l'organe creux est pressé contre le côté intérieur mais qui, dans le même temps, n'empêche pratiquement pas l'insertion de l'organe creux dans la douille respective.
